# EUROPÄISCHE PATENTSCHRIFT

(11) **EP 1 660 489 B1**
(45) Veröffentlichungstag und Bekanntmachung des Hinweises auf die Patenterteilung: **02.07.2014**
(21) Anmeldenummer: 04764810.0
(22) Anmeldetag: 03.09.2004
(51) Int. Cl.: C07D 413/12, C07D 263/20, C07D 413/14, C07D 471/04

(54) **ZWISCHENPRODUKTE FÜR DIE HERSTELLUNG VON OXAZOLIDINON-CHINOLON HYBRIDEN**
INTERMEDIATE PRODUCTS FOR PRODUCING OXAZOLIDINONE-QUINOLONE HYBRIDS
PRODUITS INTERMEDIAIRES DESTINES A LA FABRICATION D'HYBRIDES OXAZOLIDINONE-CHINOLONE

(30) Priorität: 03.09.2003 DE 10340485
(43) Veröffentlichungstag der Anmeldung: 31.05.2006
(60) Teilanmeldung: 10011797.7
(73) Patentinhaber: Morphochem Aktiengesellschaft Für Kombinatorische Chemie, 81379 München (DE)
(72) Erfinder: HUBSCHWERLEN, Christian, F-68480 Durmenach (FR); SPECKLIN, Jean-Luc, F-68680 Kembs (FR); SURIVET, Jean, Philippe, F-68300 Saint-Louis (FR); BAESCHLIN, Daniel, K., CH-4144 Arlesheim (CH)
(74) Vertreter: Forstmeyer, Dietmar
(86) Internationale Anmeldenummer: PCT/EP2004/009858
(87) Internationale Veröffentlichungsnummer: WO 2005/023801

(56) Entgegenhaltungen:
- WO-A-00/10566
- WO-A-01/09107
- WO-A-01/46164
- WO-A-03/032962
- WO-A-2004/069816
- WO-A1-2004/096221
- WO-A2-2005/058888
- US-A1- 2004 132 764
- HUBSCHWERLEN CHRISTIAN ET AL: "Structure-activity relationship in the oxazolidinone-quinolone hybrid series: Influence of the central spacer on the antibacterial activity and the mode of action." BIOORGANIC & MEDICINAL CHEMISTRY LETTERS, Bd. 13, Nr. 23, 1. Dezember 2003 (2003-12-01), Seiten 4229-4233, XP002308675 ISSN: 0960-894X

## Beschreibung

Die vorliegende Erfindung beschreibt Zwischenprodukte (ZP) für eine neue und effiziente Synthese von Endprodukten, bei denen die Pharmakophore von Chinolon und Oxazolidinon über einen chemisch stabilen Linker miteinander verknüpft sind. Endprodukte dieser Art sind in WO 03032962 beschrieben und zeichnen sich durch eine hohe Wirksamkeit gegenüber menschlichen und tierischen Bakterien aus. Des weiteren betrifft die vorliegende Erfindung eine neue und effiziente Synthese dieser Zwischenprodukte sowie der Endprodukte

Die vorliegende Erfindung betrifft Verbindungen der Formel (XI) 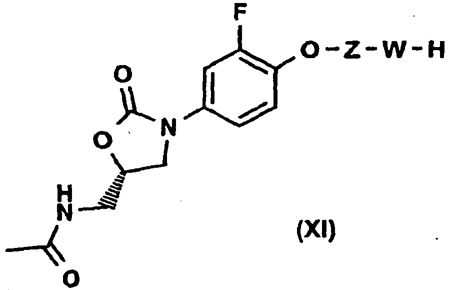 wobei
W eine durch eine OH-Gruppe substituierte Heterocyclo-alkylengruppe ist, die einen Ring mit 4, 5, 6 oder 7 Ringatomen enthält und wobei das an die Gruppe W gebundene Wasserstoffatom an ein Stickstoffatom gebunden ist; und
Z eine C₁₋₄ Alkylengruppe ist.

Der Ausdruck Alkyl bezieht sich auf eine gesättigte, geradkettige oder verzweigte Kohlenwasserstoffgruppe, die 1 bis 20 Kohlenstoffatome, vorzugsweise 1 bis 12 Kohlenstoffatome, besonders bevorzugt 1 bis 6 Kohlenstoffatome aufweist, z.B. die Methyl-, Ethyl-, Propyl-, Isopropyl-, n-Butyl-, Isobutyl-, tert-Butyl, n-Pentyl-, n-Hexyl- oder 2,2-Dimethylbutyl-Gruppe.

Die Ausdrücke Alkenyl und Alkinyl beziehen sich auf zumindest teilweise ungesättigte, geradkettige oder verzweigte Kohlenwasserstoffgruppen, die 2 bis 20 Kohlenstoffatome, vorzugsweise 2 bis 12 Kohlenstoffatome, besonders bevorzugt 2 bis 6 Kohlenstoffatome aufweisen, z. B. die Ethenyl-, Allyl-, Acetylenyl-, Propargyl-, Isoprenyl- oder Hex-2-enyl-Gruppe. Bevorzugt weisen Alkenylgruppen eine oder zwei (besonders bevorzugt eine) Doppelbindungen bzw. Alkinylgruppen eine oder zwei (besonders bevorzugt eine) Dreifachbindungen auf.

Des weiteren beziehen sich die Begriffe Alkyl, Alkenyl und Alkinyl auf Gruppen, bei der ein oder mehrere Wasserstoffatome unabhängig voneinander durch ein Halogenatom (bevorzugt F oder Cl) ersetzt sind wie z. B. die 2,2,2-Trichlorethyl-, oder die Trifluormethylgruppe.

Der Ausdruck Heteroalkyl bezieht sich auf eine Alkyl-, eine Alkenyl- oder eine Alkinyl-Gruppe (z. B. Heteroalkenyl, Heteroalkinyl), in der ein oder mehrere (bevorzugt 1, 2 oder 3) Kohlenstoffatome unabhängig voneinander durch ein Sauerstoff-, Stickstoff-, Phosphor-, Bor-, Selen-, Silizium- oder Schwefelatom ersetzt sind (bevorzugt Sauerstoff, Schwefel oder Stickstoff). Der Ausdruck Heteroalkyl bezieht sich des weiteren auf eine Carbonsäure oder eine von einer Carbonsäure abgeleitete Gruppe wie z. B. Acyl, Acylalkyl, Alkoxycarbonyl, Acyloxy, Acyloxyalkyl, Carboxyalkylamid oder Alkoxycarbonyloxy.

Beispiele für Heteroalkylgruppen sind Gruppen der Formeln R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}- , R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-. R^{a}-N (R^{b}) -CO-N (R^{c})-Y^{a}-, R^{a}-O-CO-O-Y^{a}- , R^{a}-N(R^{b}) -C (=NR^{d}) -N (R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N (R^{b}) -CS-Y^{a}-, R^{a}-O-CS-N (R^{b}) -Y^{a}-, R^{a}-N(R^{b})-CS-O-Y^{a}-, R^{a}-N (R^{b}) -CS-N(R^{c}) -Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b}) -Y^{a}-, R^{a}-N(R^{b})-CS-S-Y^{a}-, R^{a}-S-CS-O-Y^{a}-, R^{a}-O-CS-S-Y^{a}-, wobei R^{a} ein Wasserstoffatom, eine C₁-C₆-Alkyl-, eine C₂-C₆-Alkenyl- oder eine C₂-C₆-Alkinylgruppe; R^{b} ein Wasserstoffatom, eine C₁-C₆-Alkyl-, eine C₂-C₆-Alkenyl- oder eine C₃-C₆-Alkinylgruppe; R^{c} ein Wasserstoffatom, eine C₁-C₆-Alkyl-, eine C₂-C₆-Alkenyl- oder eine C₂-C₆-Alkinylgruppe; R^{d} ein Wasserstoffatom, eine C₁-C₆-Alkyl-, eine C₂-C₆-Alkenyl- oder eine C₂-C₆-Alkinylgruppe und Y^{a} eine direkte Bindung, eine C₁-C₆-Alkylen-, eine C₂-C₆-Alkenylen- oder eine C₂-C₆-Alkinylengruppe ist, wobei jede Heteroalkylgruppe mindestens ein Kohlenstoffatom enthält und ein oder mehrere Wasserstoffatome durch Fluor- oder Chloratome ersetzt sein können. Konkrete Beispiele für Heteroalkylgruppen sind Methoxy, Trifluormethoxy, Ethoxy, n-Propyloxy, iso-Propyloxy, tert-Butyloxy, Methoxymethyl, Ethoxymethyl, Methoxyethyl, Methylamino, Ethylamino, Dimethylamino, Diethylamino, iso-Propylethylamino, Methylaminomethyl, Ethylaminomethyl, Di-iso-Propylaminoethyl, Enolether, Dimethylaminomethyl, Dimethylaminoethyl, Acetyl, Propionyl, Butyryloxy, Acetyloxy, Methoxycarbonyl, Ethoxycarbonyl, N-Ethyl-N-Methylcarbamoyl oder N-Methylcarbamoyl. Weitere Beispiele für Heteroalkylgruppen sind Nitril-, Isonitril, Cyanat-, Thiocyanat-, Isocyanat-, Isothiocyanat und Alkylnitril-gruppen. Ein Beispiel für eine Heteroalkylengruppe ist eine Gruppe der Formel -CH₂CH(OH)-.

Der Ausdruck Cycloalkyl bezieht sich auf eine gesättigte oder teilweise ungesättigte (z. B. eine cyclische Gruppe, die eine, zwei oder mehrere Doppelbindungen aufweist, wie eine Cycloalkenylgruppe) cyclische Gruppe, die einen oder mehrere Ringe (bevorzugt 1 oder 2) aufweist, die 3 bis 14 Ringkohlenstoffatome, vorzugsweise 3 bis 10 (insbesondere 3, 4, 5, 6 oder 7) Ringkohlenstoffatome enthalten. Der Ausdruck Cycloalkyl bezieht sich weiterhin auf entsprechende Gruppen, bei denen ein oder mehrere Wasserstoffatome unabhängig voneinander durch Fluor-, Chlor-, Brom- oder Jodatome oder OH, =O, SH, =S, NH₂, =NH oder NO₂-Gruppen ersetzt sind also z. B. cyclische Ketone wie z. B. Cyclohexanon, 2-Cyclohexenon oder Cyclopentanon. Weitere konkrete Beispiele für Cycloalkylgruppen sind die Cyclopropyl-, Cyclobutyl-, Cyclopentyl-, Spiro[4,5]-decanyl-, Norborny-, Cyclohexyl-, Cyclopentenyl-, Cyclohexadienyl-, Decalinyl-, Bicyclo[4.3.0]nonyl-, Tetralin-, Cyclopentylcyclohexyl-, Fluorcyclohexyl- oder die Cyclohex-2-enyl-Gruppe.

Der Ausdruck Heterocycloalkyl bezieht sich auf eine Cycloalkylgruppe wie oben definiert, in der ein oder mehrere (bevorzugt 1, 2 oder 3) Ringkohlenstoffatome unabhängig voneinander durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heterocycloalkylgruppe 1 oder 2 Ringe mit 3 bis 10 (insbesondere 3, 4, 5, 6 oder 7) Ringatomen. Der Ausdruck Heterocycloalkyl bezieht sich weiterhin auf Gruppen, bei denen ein oder mehrere Wasserstoffatome unabhängig voneinander durch Fluor-, Chlor-, Brom- oder Jodatome oder OH, =O, SH, =S, NH₂, =NH oder NO₂-Gruppen ersetzt sind. Beispiele sind die Piperidyl-, Piperazinyl-, Morpholinyl-, Urotropinyl-, Pyrrolidinyl-, Tetrahydrothiophenyl-, Tetrahydropyranyl-, Tetrahydrofuryl- oder 2-Pyrazolinyl-Gruppe sowie Lactame, Lactone, cyclische Imide und cyclische Anhydride.

Der Ausdruck Alkylcycloalkyl bezieht sich auf Gruppen, die entsprechend den obigen Definitionen sowohl Cycloalkyl- wie auch Alkyl-, Alkenyl- oder Alkinylgruppen enthalten, z. B. Alkylcycloalkyl-, Cycloalkylalkyl-, Alkylcycloalkenyl-, Alkenylcycloalkyl- und Alkinylcycloalkylgruppen. Bevorzugt enthält eine Alkylcycloalkylgruppe eine Cycloalkylgruppe, die einen oder zwei Ringsysteme aufweist, welche 3 bis 10 (insbesondere 3, 4, 5, 6 oder 7) Kohlenstoffatome enthalten und eine oder zwei Alkyl-, Alkenyl- oder Alkinylgruppen mit 1 oder 2 bis 6 Kohlenstoffatomen.

Der Ausdruck Heteroalkylcycloalkyl bezieht sich auf Alkylcycloalkylgruppen, wie oben definiert, in der ein oder mehrere (bevorzugt 1, 2 oder 3) Kohlenstoffatome unabhängig voneinander durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heteroalkylcycloalkylgruppe 1 oder 2 Ringsysteme mit 3 bis 10 (insbesondere 3, 4, 5, 6 oder 7) Ringatomen und eine oder zwei Alkyl, Alkenyl, Alkinyl oder Heteroalkylgruppen mit 1 oder 2 bis 6 Kohlenstoffatomen. Beispiele derartiger Gruppen sind Al-kylheterocycloalkyl, Alkylheterocycloalkenyl, Alkenylheterocycloalkyl, Alkinylheterocycloalkyl, Heteroalkylcycloalkyl, Heteroalkylheterocycloalkyl und Heteroalkylheterocylcloalkenyl, wobei die cyclischen Gruppen gesättigt oder einfach, zweifach oder dreifach ungesättigt sind.

Der Ausdruck Aryl bzw. Ar bezieht sich auf eine aromatische Gruppe, die einen oder mehrere Ringe aufweist, und 6 bis 14 Ringkohlenstoffatome, vorzugsweise 6 bis 10 (insbesondere 6) Ringkohlenstoffatome enthält. Der Ausdruck Aryl (bzw. Ar) bezieht sich weiterhin auf Gruppen, bei denen ein oder mehrere Wasserstoffatome unabhängig voneinander durch Fluor-, Chlor-, Brom- oder Jodatome oder OH, SH, NH₂, oder NO₂-Gruppen ersetzt sind. Beispiele sind die Phenyl-, Naphthyl-, Biphenyl-, 2-Fluorphenyl, Anilinyl-, 3-Nitrophenyl oder 4-Hydroxyphenyl-Gruppe.

Der Ausdruck Heteroaryl bezieht sich auf eine aromatische Gruppe, die einen oder mehrere Ringe aufweist, und 5 bis 14 Ringatome, vorzugsweise 5 bis 10 (insbesondere 5 oder 6) Ringatome enthält und ein oder mehrere (bevorzugt 1, 2, 3 oder 4) Sauerstoff-, Stickstoff-, Phosphor- oder Schwefel-Ringatome (bevorzugt O, S oder N) enthält. Der Ausdruck Heteroaryl bezieht sich weiterhin auf Gruppen, bei denen ein oder mehrere Wasserstoffatome unabhängig voneinander durch Fluor-, Chlor-, Brom- oder Jodatome oder OH, SH, NH₂, oder NO₂-Gruppen ersetzt sind. Beispiele sind 4-Pyridyl-, 2-Imidazolyl-, 3-Phenylpyrrolyl-, Thiazolyl-, Oxazolyl-, Triazolyl-, Tetrazolyl-, Isoxazolyl-, Indazolyl-, Indolyl-, Benzimidazolyl-, Pyridazinyl-, Chinolinyl-, Purinyl-, Carbazolyl-, Acridinyl-, Pyrimidyl-, 2,3'-Bifuryl-, 3-Pyrazolyl- und Isochinolinyl-Gruppen.

Der Ausdruck Aralkyl bezieht sich auf Gruppen, die entsprechend den obigen Definitionen sowohl Aryl- als auch Alkyl-, Alkenyl-, Alkinyl- und/oder Cycloalkylgruppen enthalten, wie z. B. Arylalkyl-, Arylalkenyl-, Arylalkinyl-, Arylcycloalkyl-, Arylcycloalkenyl-, Alkylarylcycloalkyl- und Alkylarylcycloalkenylgruppen. Konkrete Beispiele für Aralkyle sind Toluol, Xylol, Mesitylen, Styrol, Benzylchlorid, o-Fluortoluol, 1H-Inden, Tetralin, Dihydronaphthaline, Indanon, Phenylcyclopentyl, Cumol, Cyclohexylphenyl, Fluoren und Indan. Bevorzugt enthält eine Aralkylgruppe ein oder zwei aromatische Ringsysteme (1 oder 2 Ringe) mit 6 bis 10 Kohlenstoffatomen und ein oder zwei Alkyl-, Alkenyl- und/oder Alkinylgruppen mit 1 oder 2 bis 6 Kohlenstoffatomen und/oder eine Cycloalkylgruppe mit 5 oder 6 Ringkohlenstoffatomen.

Der Ausdruck Heteroaralkyl bezieht sich auf eine Aralkyl-gruppe wie oben definiert, in der ein oder mehrere (bevorzugt 1, 2, 3 oder 4) Kohlenstoffatome unabhängig voneinander durch ein Sauerstoff-, Stickstoff-, Silizium-, Selen-, Phosphor-, Bor-oder Schwefelatom (bevorzugt Sauerstoff, Schwefel oder Stickstoff) ersetzt sind, d. h. auf Gruppen, die entsprechend den obigen Definitionen sowohl Aryl- bzw. Heteroaryl- wie auch Alkyl-, Alkenyl-, Alkinyl- und/oder Heteroalkyl- und/oder Cycloalkyl- und/oder Heterocyclo-alkylgruppen enthalten. Bevorzugt enthält eine Heteroaralkylgruppe ein oder zwei aromatische Ringsysteme (1 oder 2 Ringe) mit 5 oder 6 bis 10 Ringkohlenstoffatomen und ein oder zwei Alkyl-, Alkenyl-und/oder Alkinylgruppen mit 1 oder 2 bis 6 Kohlenstoffatomen und/oder eine Cycloalkylgruppe mit 5 oder 6 Ringkohlenstoffatomen, wobei 1, 2, 3 oder 4 dieser Kohlenstoffatome unabhängig voneinander durch Sauerstoff-, Schwefel- oder Stickstoffatome ersetzt sind.

Beispiele sind Arylheteroalkyl-, Arylheterocycloalkyl-, Arylheterocycloalkenyl-, Arylalkylheterocycloalkyl-, Arylalkenylheterocycloalkyl-, Arylalkinylheterocycloalkyl-, Arylalkylheterocycloalkenyl-, Heteroarylalkyl-, Heteroarylalkenyl-, Heteroarylalkinyl-, Heteroarylheteroalkyl-, Heteroarylcycloalkyl-, Heteroarylcycloalkenyl-, Heteroarylheterocycloalkyl-, Heteroarylheterocycloalkenyl-, Heteroarylalkylcycloalkyl-, Heteroarylalkylheterocycloalkenyl-, Heteroarylheteroalkylcycloalkyl-, Heteroarylheteroalkylcycloalkenyl- und Heteroarylheteroalkylheterocycloalkyl-Gruppen, wobei die cyclischen Gruppen gesättigt oder einfach, zweifach oder dreifach ungesättigt sind. Konkrete Beispiele sind die Tetrahydroisochinolinyl-, Benzoyl-, 2- oder 3-Ethylindolyl-, 4-Methylpyridino-, 2-, 3- oder 4-Methoxyphenyl-, 4-Ethoxyphenyl-, 2-, 3- oder 4-Carboxyphenylalkylgruppe.

Die Ausdrücke Alkyl, Alkenyl, Alkinyl, Heteroalkyl, Cycloalkyl, Heterocycloalkyl, Alkylcyclo-alkyl, Heteroalkylcycloalkyl, Aryl, Heteroaryl, Aralkyl und Heteroaralkyl beziehen sich auch auf Gruppen, in denen ein oder mehrere Wasserstoffatome solcher Gruppen unabhängig voneinander durch Fluor-, Chlor-, Brom- oder Jodatome oder OH, =O, SH, =S, NH₂, =NH oder NO₂-Gruppen ersetzt sind.

Der Ausdruch "gegebenenfalls substituiert" bezieht sich auf Gruppen, in denen ein oder mehrere Wasserstoffatome unabhängig voneinander durch Fluor-, Chlor-, Brom- oder Jodatome oder OH, =O, SH, =S, NH₂, =NH oder NO₂-Gruppen ersetzt sind. Dieser Ausdruck bezieht sich weiterhin auf Gruppen, die mit unsubstituierten C₁-C₆ Alkyl-, C₂-C₆ Alkenyl-, C₂-C₆ Alkinyl-, C₁-C₆ Heteroalkyl-, C₃-C₁₀ Cycloalkyl-, C₂-C₉ Heterocycloalkyl-, C₆-C₁₀ Aryl-, C₁-C₉ Heteroaryl-, C₇-C₁₂ Aralkyl- oder C₂-C₁₁ Heteroaralkyl-Gruppen substituiert sind.

Die in der vorliegenden Anmeldung beschriebenen Verbindungen können aufgrund ihrer Substitution ein oder mehrere Chiralitätszentren enthalten. Die vorliegende Erfindung umfasst daher sowohl alle reinen Enantiomere und alle reinen Diastereomere, als auch deren Gemische in jedem Mischungsverhältnis. Des weiteren sind von der vorliegenden Erfindung auch alle cis/trans-Isomeren der Verbindungen der allgemeinen Formel (I) sowie Gemische davon umfasst. Des weiteren sind von der vorliegenden Erfindung alle tautomeren Formen der beschriebenen Verbindungen umfasst.

Insbesondere bevorzugt ist Z eine CH₂ oder eine CH₂CH₂ Gruppe.

Besonders bevorzugt sind Z-W zusammen eine Gruppe der Formel: 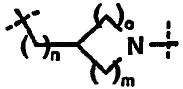 wobei n gleich 1 oder 2, m gleich 1 oder 2 und o gleich 1 oder 2 ist wobei diese Gruppe durch eine OH Gruppe substituiert ist.

Besonders bevorzugt weist W die folgende Struktur auf: 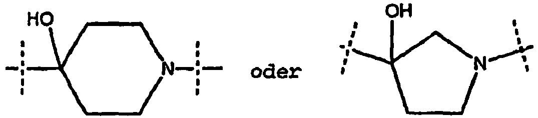 Verbindungen der Formel (XI) können bei der Synthese von Verbindungen der Formel (I) 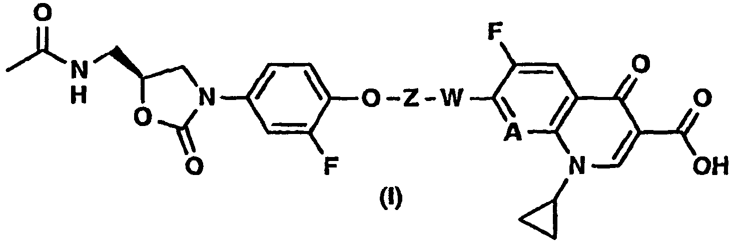 verwendet werden,
wobei Z eine gegebenenfalls substituierte C₁₋₄ Alkylengruppe, A ein Stickstoffatom oder eine CH-Gruppe und W eine durch eine OH-Gruppe substituierte Heterocycloalkylengruppe ist, welche mindestens ein Stickstoffatom enthält und wobei der Chinolinrest an dieses Stickstoffatom gebunden ist.

Verbindungen der Formel (I) können wie folgt hergestellt werden: 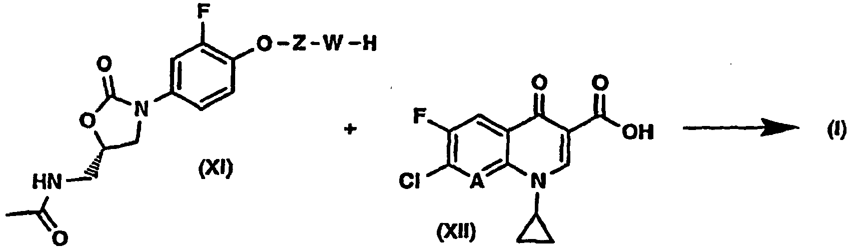 wobei Verbindung (XI) eine Verbindung gemäß der vorliegenden Erfindung ist und Verbindung (XII) bevorzugt in Form eines BorKomplexes (z.B. als Bordiacetat-Komplex) eingesetzt wird.

Die bevorzugten Reaktionsbedingungen für diesen Schritt sind: N-Methylpyrrolidon, Trimethylsilylchlorid, Hünig Base oder K₂CO₃, 80°C.

Erfindungsgemäße Verbindungen können z.B. über folgenden Syntheseweg hergestellt werden:

### Stufe 1:

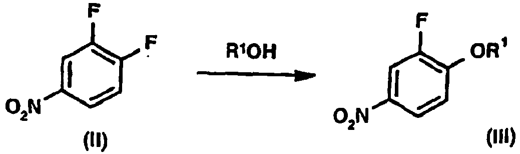

### Stufe 2:

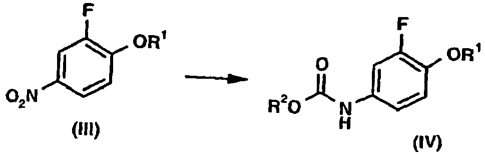

### Stufe 3:

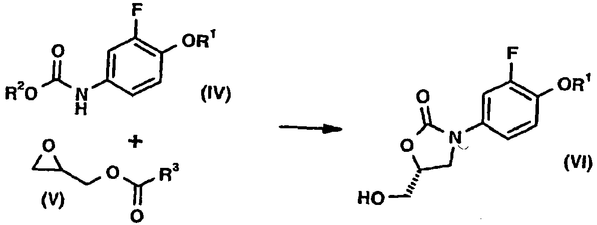

### Stufe 4:

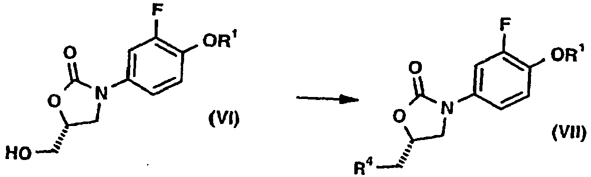

### Stufe 5:

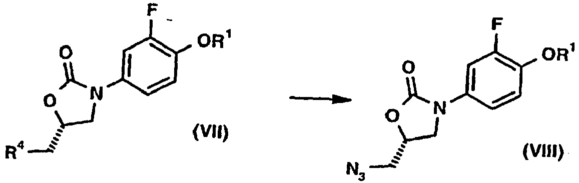

### Stufe 6:

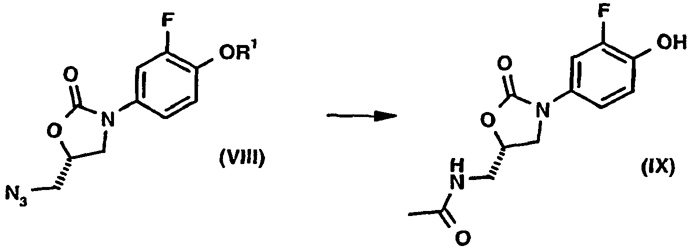

### Stufe 7:

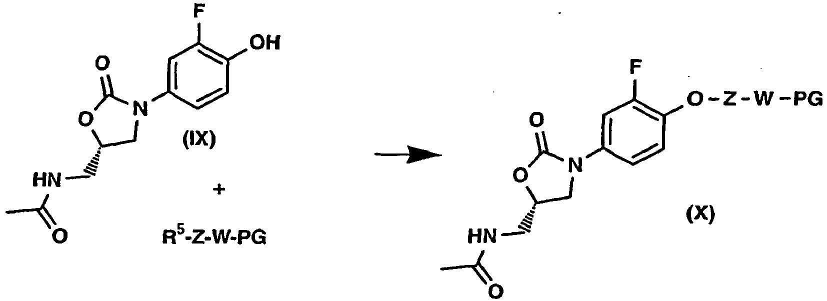

### Stufe 8:

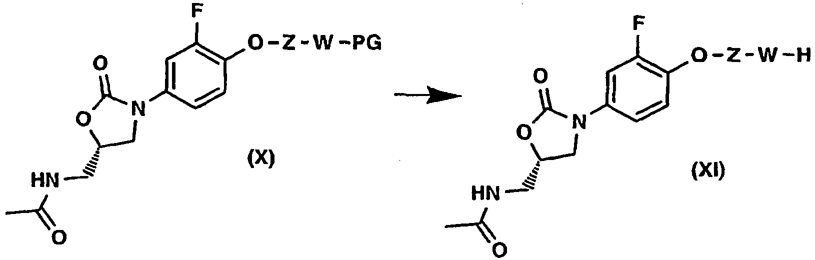

Alternativ können Verbindungen der Formel (ZP) bzw. (XI) durch folgenden Syntheseweg hergestellt werden:

### Stufe 1:

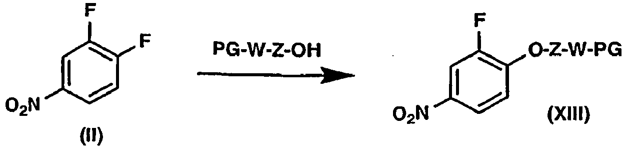

### Stufe 2:

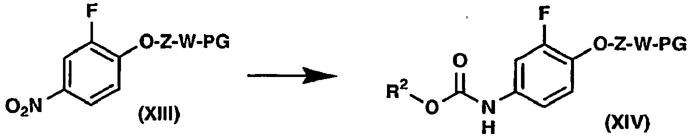

### Stufe 3:

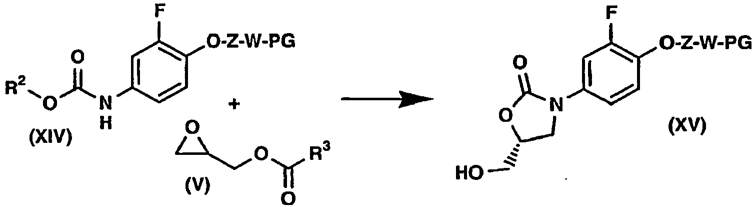

### Stufe 4:

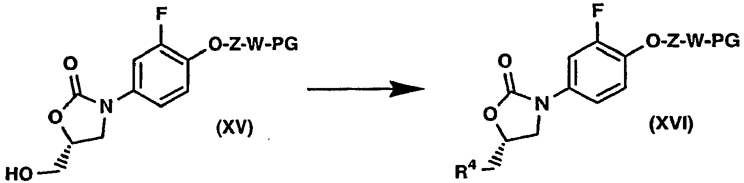

### Stufe 5:

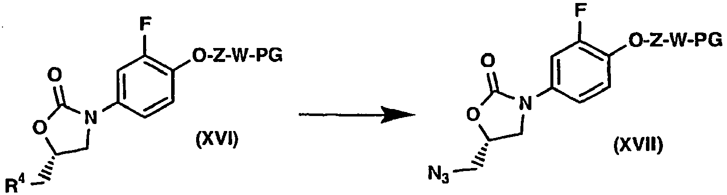

### Stufe 6:

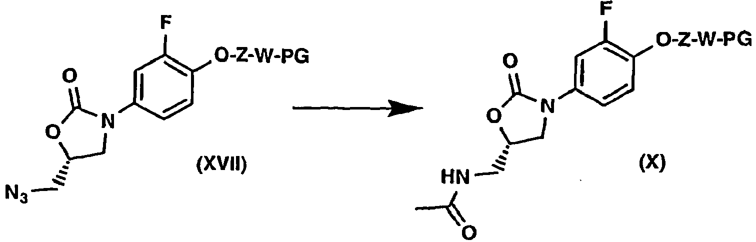

### Stufe 7:

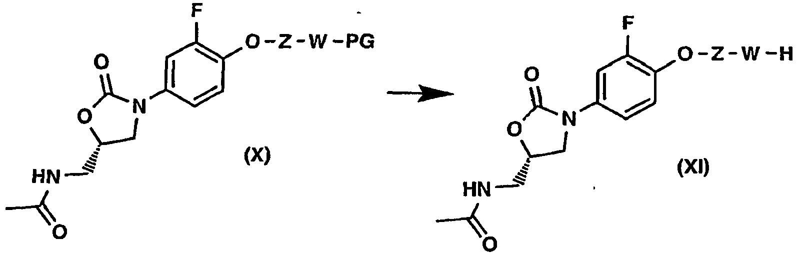

Wenn bei diesem Syntheseweg als Schutzgruppe PG die Cbz-Schutzgruppe gewählt wird, entfällt Stufe 7, da dann bei Stufe 6 direkt Verbindung (XI) entsteht.

Dabei sind:
PG eine an sich übliche Schutzgruppe für Amine; insbesondere eine Benzyloxycarbonyl- (Cbz-) Gruppe;
R¹ eine gegebenenfalls substituierte Benzyl- (z. B. p-Methoxy-benzyl) oder Allylgruppe;
R² eine C₁₋₄ Alkyl, eine Allyl oder eine Benzylgruppe;
R³ eine C₁₋₄ Alkylgruppe;
R⁴ eine Mesyloxy-, Tosyloxy-, Triflyloxy- oder Texyloxygruppe oder ein Chlor-, Brom- oder Iodatom und
R⁵ eine Mesyloxy-, Tosyloxy-, Triflyloxy- oder Texyloxygruppe oder ein Chlor-, Brom- oder Iodatom.

Schutzgruppen sind dem Fachmann bekannt und z. B. in P. J. Kocienski, Protecting Groups, Georg Thieme Verlag, Stuttgart, 1994 sowie in T. W. Greene, P. G. M. Wuts, Protective Groups in Organic Synthesis, John Wiley & Sons, New York, 1999 beschrieben. Gängige Aminoschutzgruppen sind z. B. t-Butyloxycarbonyl- (Boc), Benzyloxycarbonyl- (Cbz, Z), Benzyl-(Bn), Benzoyl- (Bz), Fluorenylmethyloxycarbonyl- (Fmoc), Allyloxycarbonyl-(Alloc), Trichlorethyloxycarbonyl- (Troc), Acetyl- oder Trifluoracetylgruppen.

Wiederum bevorzugt ist R¹ eine Benzylgruppe.

Des weiteren bevorzugt ist R² eine Benzylgruppe.

Weiter bevorzugt ist R³ eine n-Propylgruppe.

Wiederum bevorzugt ist R⁴ eine Mesyloxygruppe.

Des weiteren bevorzugt ist R⁵ eine Mesyloxygruppe.

Bevorzugte Reaktionsbedingungen für den ersten Syntheseweg sind:
- Für Stufe 1:: CH₂Cl₂, Kaliumhydroxid, Raumtemperatur;
- Für Stufe 2:: Wasserstoff/Pt/C; anschliessend Cbz-Cl, NaHCO₃, Aceton/Wasser; beides bei Raumtemperatur;
- Für Stufe 3:: (R)-Glycidylbutyrat (V), n BuLi, -60°C bzw. LDA, -15°C;
- Für Stufe 4:: Methylsulfonylchlorid, Triethylamin, CH₂Cl₂;
- Für Stufe 5:: NaN₃ in DMF, katalytische Mengen Bu₄NI, 90°C;
- Für Stufe 6:: Wasserstoff/Pd(OH)₂, THF, MeOH; anschliessend AcOH, Ac₂O; beides bei Raumtemperatur;
- Für Stufe 7:: Dimethylformamid (DM_{F}),Natriumhydrid, 70°C;
- Für Stufe 8:: H₂/Pd (OH)₂, THF, Methanol, Raumtemperatur;

Bevorzugte Reaktionsbedingungen für den zweiten Syntheseweg sind:
- Für Stufe 1:: Mitsunobu Reaktion oder Base (z.B. NaH), DMF, Tosylat von PG-W-Z-OH;
- Für Stufe 2:: Wasserstoff/Pt/C; anschliessend Cbz-Cl, NaHCO₃, Aceton/Wasser; beides bei Raumtemperatur oder Sn, HCl;
- Für Stufe 3:: (R)-Glycidylbutyrat (V), n BuLi, -60°C bzw. LDA, -15°C;
- Für Stufe 4:: Methylsulfonylchlorid, Triethylamin, CH₂Cl₂;
- Für Stufe 5:: NaN₃ in DMF, katalytische Mengen Bu₄NI, 90°C;
- Für Stufe 6:: Wasserstoff/Pd(OH)₂, THF, MeOH; anschliessend AcOH, Ac₂O; beides bei Raumtemperatur;

In den folgenden Beispielen wird die Synthese von Verbindungen der Formel (XI) sowie deren Verwendung zur Synthese von Verbindungen der Formel (I) beschrieben.

### Beispiele

### Beispiel 1:

### 7-(4-{4-[(5S)-5-(Acetylaminomethyl)-2-oxo-oxazolidin-3-yl]-2-fluorphenoxymethyl}-4-hydroxypiperidin-1-yl)-1-cyclopropyl-6-fluor-4-oxo-1,4-dihydrochinolin-3-carbonsäure:

### Stufe 1: (4-Benzyloxy-3-fluorphenyl)-carbaminsäurebenzylester:

Eine Mischung aus 34.9g 1-Benzyloxy-2-fluor-4-nitrobenzol (WO03064413) (MW:247.28, 141mmol) und 340mg Platin (5% auf Aktivkohle) in 350ml Essigester wurde unter einer Wasserstoffatmosphäre bei RT und Normaldruck gerührt. Der Reaktionsverlauf wurde mittels HPLC verfolgt und die Reaktion war nach 20h beendet. Der Katalysator wurde abfiltriert und das Filtrat unter reduziertem Druck zur Trockene einrotiert. Der ölige Ruckstand wurde in 500ml Aceton gelöst und mit 250ml einer gesättigten Natriumhydrogencarbonatlösung und 17.5g Natriumhydrogencarbonat (MW: 84.01, 208mmol) versetzt. Die Mischung wurde auf 5°C gekühlt und 26.08g Benzylchloroformiat (MW:170.59, 152mmol) zugetropft. Die Mischung wurde anschliessend 2h bei RT gerührt und der Reaktionsverlauf mittels DC (Hexan/Essigester 3:1) verfolgt. Das Aceton wurde unter vermindertem Druck entfernt, der Rückstand mit 500ml Wasser versetzt, und der Feststoff abfiltriert. Die Kristalle wurden mit 500ml Wasser gewaschen und getrocknet.
Ausbeute: 48.05g, 95.8%. MS: 352.5 (M+H)⁺, 350.8, (M-H)⁻. Methode: ESI⁺, ESI⁻.

### Stufe 2: (5R)-3-(4-benzyloxy-3-fluorphenyl)-5-hydroxymethyl-oxazolidin-2-on:

Eine gerührte Lösung von 17.5g (4-Benzyloxy-3-fluorphenyl)-carbaminsäurebenzylester (MW: 351.38, 50mmol) in 30ml trockenem Tetrahydrofuran wurde mit einem Trockeneis/ Aceton-Bad auf-78°C gekühlt. 22.8ml einer 2.3M n-Butyllithium Lösung in n-Hexan (52.5mol) wurden zugetropft und die Reaktionsmischung wurde bei -78°C für 15 min gerührt. 7.92g *R(-)*-Glycidylbutyrat (MW: 144.17, 60mmol) wurden zugegeben und die Reaktionsmischung auf RT erwärmt. Die Reaktion wurde mittels HPLC verfolgt, anschließend mit einer gesättigten Ammoniumchloridlösung gequenched und mit 100ml Essigester verdünnt. Die organische Phase wurde mit 200ml Wasser und 200ml gesättigter Kochsalzlösung gewaschen. Die organische Phase wurde über Magnesiumsulfat getrocknet, filtriert und das Filtrat unter vermindertem Druck eingeengt. Der Rückstand wurde aus 200ml Essigester/Hexan (1/1) kristallisiert. Der erhaltene Feststoff wurde aus 150ml Essigester/Dichlormethan (9/1) rekristallisiert. Die farblosen Kristalle wurden gesammelt und getrocknet. Ausbeute: 10.4g, 65.5%. MS: 318.1 (M+H)+. Methode: ESI⁺.

### Stufe 3: (5S)-5-Azidomethyl-3-(4-benzyloxy-3-fluorphenyl)-oxazolidin-2-on:

Eine Mischung aus 10g (5R)-3-(4-Benzyloxy-3-fluorphenyl)-5-hydroxymethyloxazolidin-2-on (MW: 317.32, 31.51mmol) und 4.78g Triethylamin (MW: 101.19, 47.26mmol) in 300ml Dichlormethan wurden unter Rühren bei 10°C mit 4.32g Methansulfonylchlorid (MW: 114.55, 37.82mmol) versetzt. Die Reaktionsmischung wurde bei RT für 1h gerührt und der Reaktionsverlauf mittels DC (Essigester/Hexan 1/1) verfolgt. Die Reaktion wurde mit 100ml Wasser gequenched und die organische Phase mit 100ml gesättigter Kochsalzlösung gewaschen. Die organische Phase wurde über Magnesiumsulfat getrocknet, filtriert und das Filtrat unter reduziertem Druck eingeengt. Der Rückstand wurde in 100ml Dimethylformamid gelöst und 5.12g Natriumazid (MW: 65.01, 78.7mmol) und eine katalytische Menge Tetrabutylammoniumiodid zugegeben. Die Suspension wurde bei 90°C über Nacht gerührt. Der Reaktionsverlauf wurde mittels HPLC verfolgt. Das Dimethylformamid wurde under vermindertem Druck abrotiert, der Rückstand in 200ml Dichlormethan gelöst und die organische Phase nacheinander mit 100ml Wasser und 100ml gesättigter Kochsalzlösung gewaschen. Die Dichlormethanlösung wurde über Magnesiumsulfat getrocknet, filtriert und das Filtrat unter vermindertem Druck eingeengt. Der Rückstand wurde aus 150ml Essigester/Hexan 1/1 kristallisiert. Ausbeute: 10.4g, 97%. MS: 343.1 (M+H)⁺. Methode: ESI⁺.

### Stufe 4: N-[(5S)-{3-(3-Fluor-4-hydroxyphenyl)}-2-oxo-oxazolidin-5-ylmethyl]-acetamid:

Eine Suspension von 10.4g (5S)-5-Azidomethyl-3-(4-benzyloxy-3-fluorphenyl)oxazolidin-2-on (MW: 342.33, 30.38mmol) und 1.5g Palladium (10% auf Aktivkohle) in 400ml einer 1:1 Methanol:Essigester Mischung wurde bei Raumtemperatur unter einer Wasserstoffatmosphäre für zwei Tage gerührt. Der Katalysator wurde abfiltriert und das Filtrat unter vermindertem Druck verdampft. Der Rückstand wurde in 100ml Essigsäure gelöst und mit 3.72g Essigsäureanhydrid (MW: 102.09, 36.45mmol) versetzt. Das Lösungsmittel wurde unter vermindertem Druck verdampft und der Rückstand aus einer 1:1 Essigester: Hexane Mischung rekristallisiert. Ausbeute: 6.76g, 83%. MS: 269.4 (M+H)⁺, 267.3, (M-H)⁻. Methode: ESI⁺, ESI⁻.

### Stufe 5: 4-{4-[(5S)-5-(Acetylaminomethyl)-2-oxo-oxazolidin-3-yl]-2-fluorphenoxymethyl}-4-hydroxypiperidin-1-carbonsäurebenzylester:

Eine Suspension von 22.72g 1-Oxa-6-aza-spiro[2.5]octan-6-carbonsäurebenzylester (WO9803507) (MW: 247.29, 92mmol), 21.45g N-[(5S)-{3-(3-Fluor-4-hydroxyphenyl)}-2-oxo-oxazolidin-5-yl-methyl]-acetamid (MW: 268.246, 80mmol) und 16.58g Kaliumcarbonat (MW: 138.20, 120mmol) in 150ml Dimethylformamid wurde bei 100°C für 7h gerührt. Der Reaktionsverlauf wurde mittels DC (Dichlormethan/Methanol 9:1) verfolgt. Das Dimethylformamid wurde unter vermindertem Druck verdampft und der Rückstand in 600ml einer 9:1 Mischung von Dichloromethan:Methanol gelöst. Die organische Phase wurde mit 400ml Wasser und 400ml gesättigter Kochsalzlösung gewaschen, mit Magnesiumsulfat getrocknet, filtriert und das Filtrat mit 250ml Essigester verdünnt. Die Mischung wurde unter vermindertem Druck auf ein Endvolumen von 400ml eingeengt. Die Mischung wurde bei RT über Nacht gerührt. Die Kristalle wurden anschliessend filtriert und nacheinander mit 150ml Essigester und 100ml Pentan gewaschen. Ausbeute: 31.65g, 76.7%. MS: 516.8 (M+H)⁺, Methode: ESI⁺.

### Stufe 6: N-[{(5S)-3[3-Fluor-4-(4-hydroxypiperidin-4-ylmethoxy)-phenyl]-2-oxo-oxazolidin-5-ylmethyl}]-acetamid:

Eine Suspension von 31g 4-{4-[(5S)-5-(Acetylaminomethyl)-2-oxo-oxazolidin-3-yl]-2-fluorphenoxymethyl}-4-hydroxypiperidin-1-carbonsäurebenzylester (MW: 515,54 60.13mmol) und 2.5 g Palladium (10% auf Aktivkohle) in 310ml Methanol und 150ml Essigester wurde unter einer Wasserstoffatmosphäre für 4h gerührt. Der Reaktionsverlauf wurde mittels DC (Essigester) verfolgt. Die Suspension wurde mit 300ml Methanol verdünnt, auf 40°C erwärmt, und der Katalysator über ein Glasfaser Filterpapier abfiltriert. Das Filtrat wurde auf 150ml eingeengt, mit 300ml Essigester verdünnt und wiederum auf 200ml eingeengt. 200ml Diethylether wurden zugegeben und die Suspension unter Rühren auf 0°C gekühlt. Der Feststoff wurde gesammelt und getrocknet. Ausbeute: 21.6g, 94.3%. MS: 382.6 (M+H)⁺, Methode: ESI⁺.

### Stufe 7: 7-(4-{4-[(5S)-5-(Acetylaminomethyl)-2-oxo-oxazolidin-3-yl]-2-fluorphenoxymethyl}-4-hydroxypiperidin-1-yl)-1-cyclo-propyl-6-fluor-4-oxo-1,4-dihydrochinolin-3-carbonsäure

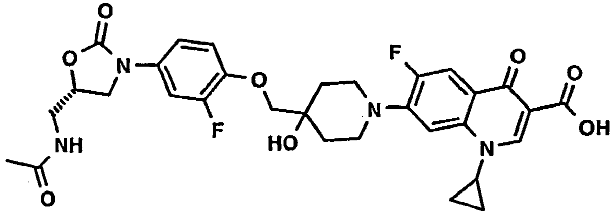

Eine Lösung von 60g N-[{(5S)-3[3-Fluor-4-(4-hydroxypiperidin-4-ylmethoxy)-phenyl]-2-oxo-oxazolidin-5-ylmethyl}]-acetamid (C₁₈H₂₄FN₃O₅, MW: 381.40 0.157 mol) und 26.87ml Ethyldiisopropylamin (MW: 129.25, 0.157 mol) in 300ml N-Methylpyrrolidin-2-on wurde mit 67.81g 7-Chlor-1-cyclopropyl-6-fluor-1,4-dihydro-4-oxo-3-chinolincarbonsäure-Bordiacetat-Komlpex (MW:410.57, 0.165 mol) versetzt und die Mischung 5h bei 80°C gerührt. Das N-Methylpyrrolidin-2-on wurde unter vermindertem Druck einrotiert und der Rückstand in 300ml Methanol gelöst. Trockener Chlorwasserstoff wurde für 30 min bei 10°C durch die Lösung geleitet. Die Lösung wurde bei RT gerührt, wobei ein gelber Feststoff ausgefallen ist. Die Umwandlung des Borkomplexes in die freie Säure wurde mittels HPLC verfolgt. Die Mischung wurde mit 300ml Essigester (Ethylacetat) verdünnt. Der Feststoff wurde abfiltriert und mit 100ml Essigester/Methanol (8/2) und 100ml Essigester gewaschen. Der gelbe Feststoff wurde getrocknet, wobei 86.4g eines gelben Feststoffes zurückblieben. Der Feststoff wurde in 200ml 24 Dimethylsulfoxid bei 40°C gelöst und die gelbe Lösung unter Rühren in 1000ml Wasser gegeben. Der gelbe Feststoff wurde gesamelt, mit Wasser gewaschen und getrocknet. Ausbeute: 73g, 74.5%. MS: 627.8 (M+H)⁺, 625.8 (M+H)⁻, Methode: ESI⁺, ESI⁻.

### Beispiel 2 (Referenzbeispiel):

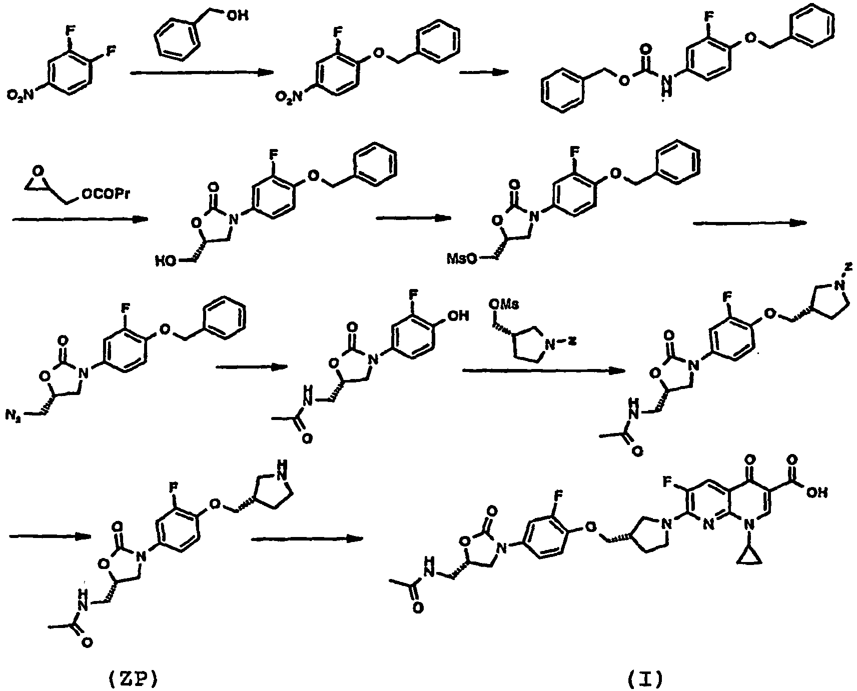

### Reaktionsbedingungen:

Stufe 1: CH₂Cl₂, KOH (50%), 3h, RT; 97%. Stufe 2: H₂, Pt/C, 20h, RT; anschliessend Cbz-Cl, Aceton/Wasser, NaHCO₃, 12h, RT, 98%. Stufe 3: n BuLi, -60°C, 24h, 80%. Stufe 4: MsCl, TEA, CH₂Cl₂; 100%. Stufe 5: NaN₃ in DMF, 90°C, kat. Bu₄NI, 5h, 90%. Stufe 6: H₂, Pd(OH)₂, THF, MeOH, 24h, anschliessend AcOH, Ac₂O, RT, 2h, 70%. Stufe 7: DMF, NaH, 70°C, 12h, 75%. Stufe 8: H₂, Pd(OH)₂, MeOH, THF, 24h, RT, 100%. Stufe 9: N-Methylpyrrolidinon, 1-Cyclopropyl-7-chloro-6-fluoro-1,4-dihydro-4-oxo-1,8-naphthydrin-3-carbonsäure (kommerziell erhältlich), TMSCl, Hünig Base oder K₂CO₃, 80°C, 5h, 80%.

Bei keiner dieser Stufen ist eine chromatographische Trennung nötig.

### Weitere Beispiele und Referenzbeispiele

Die folgenden Verbindungen wurden analog zu den oben beschriebenen Verfahren unter Verwendung von geeigneten Startmaterialien hergestellt. Bei Verbindungen, die freie OH Gruppen enthalten, wurden ferner Verbindungen hergestellt, bei denen diese OH Gruppen mit Schutzgruppen (z.B. Acetat, Benzoat, MOM-Ether oder Isopropyliden) versehen sind.

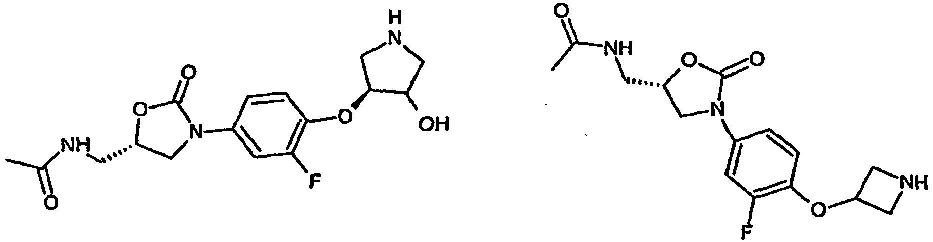

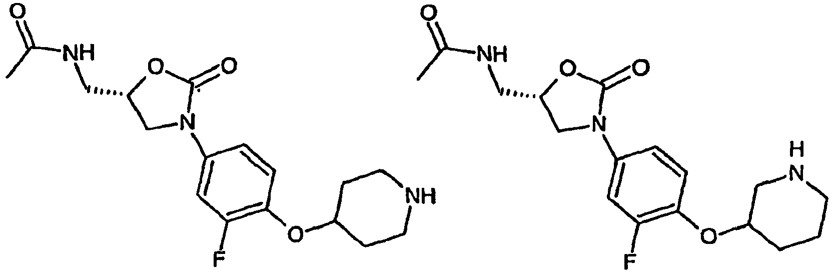

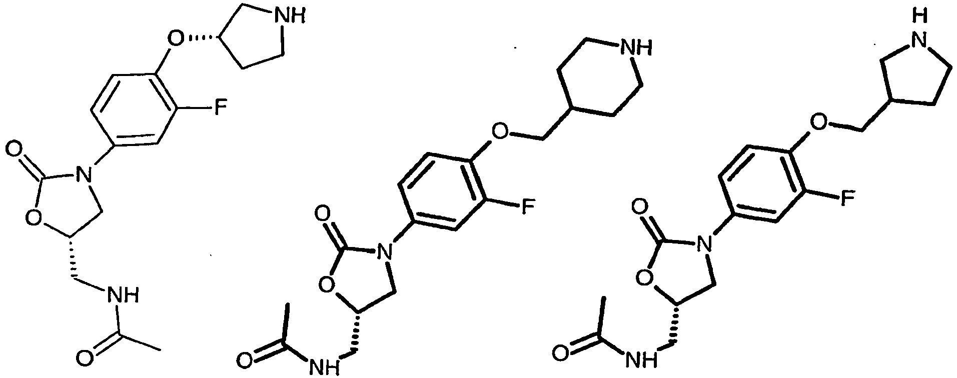

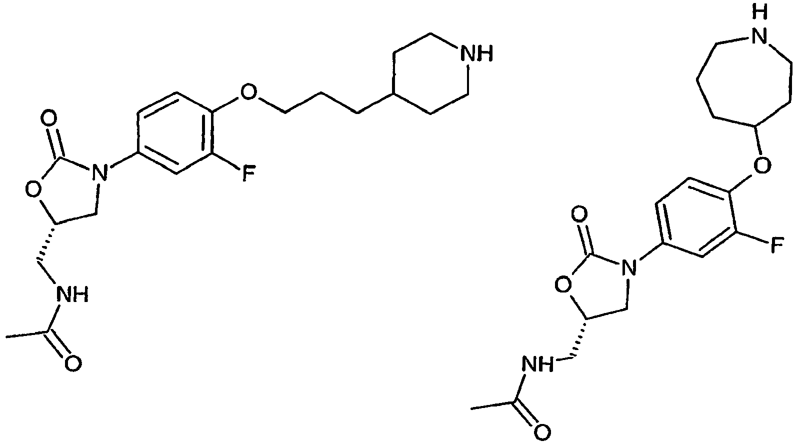

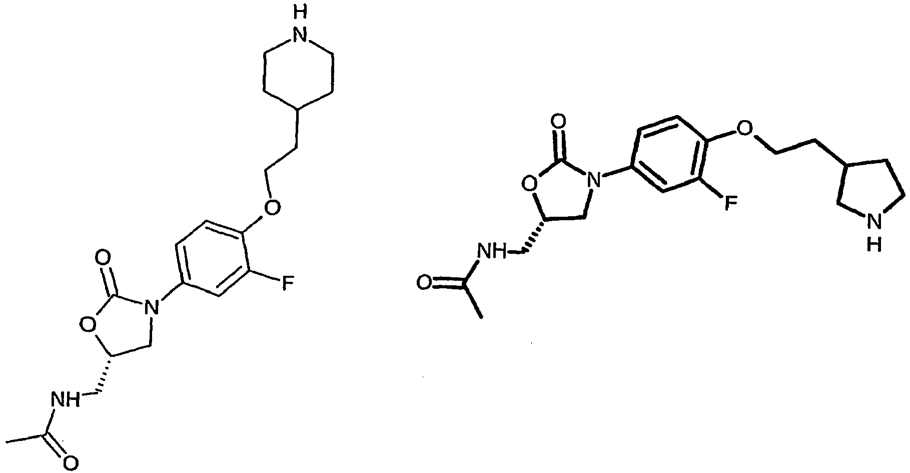

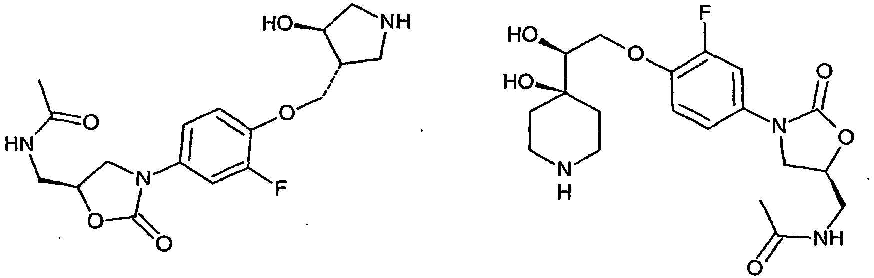

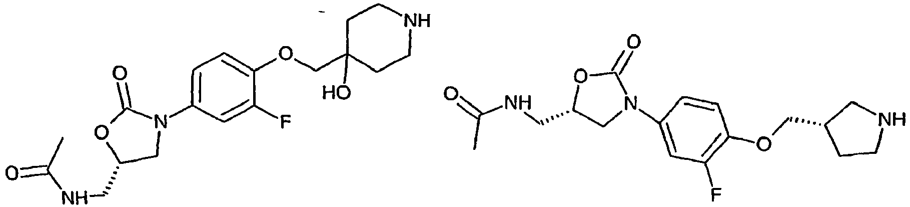

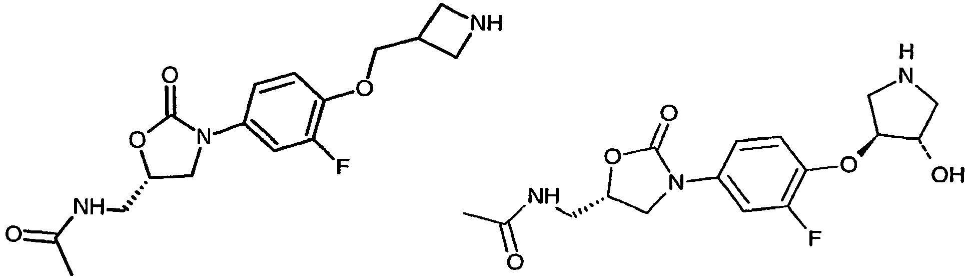

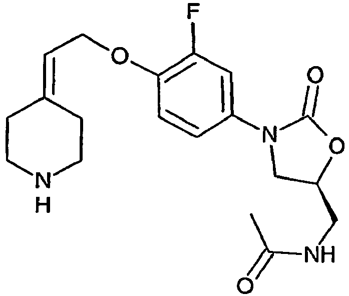

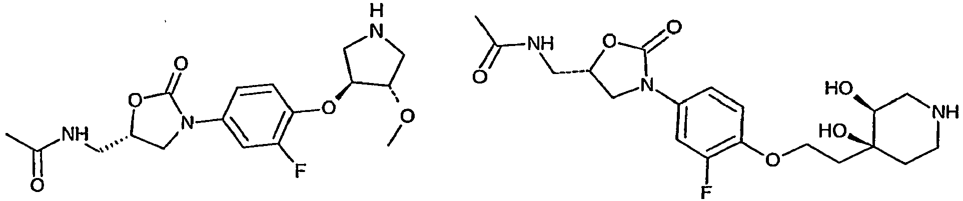

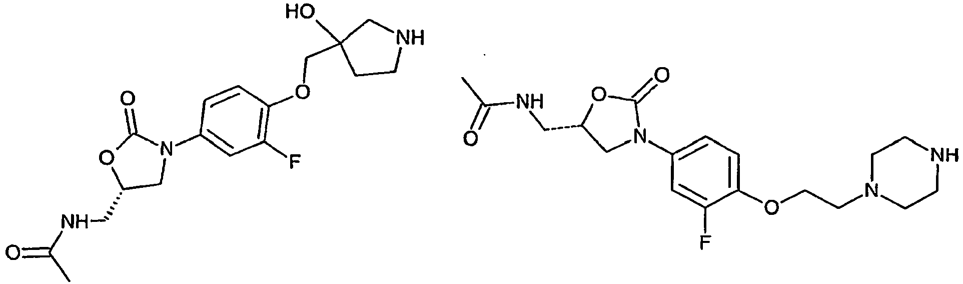

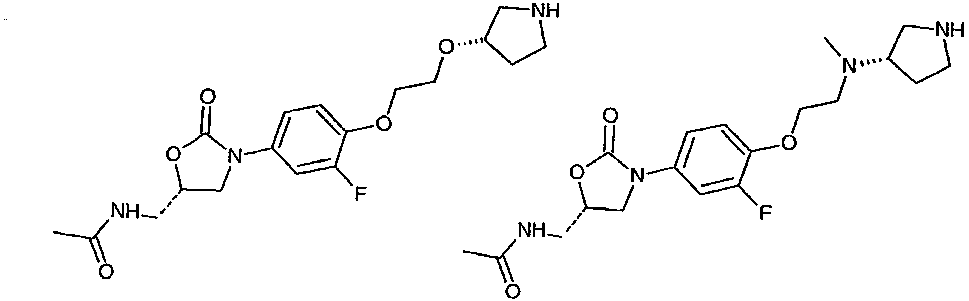

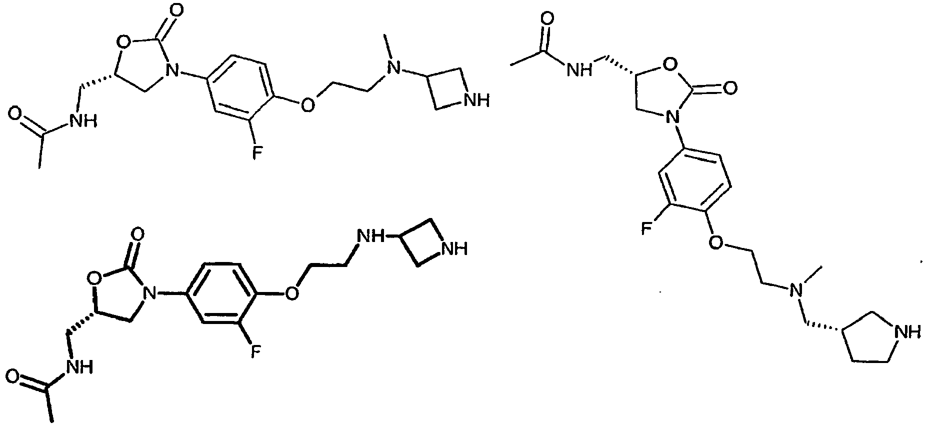

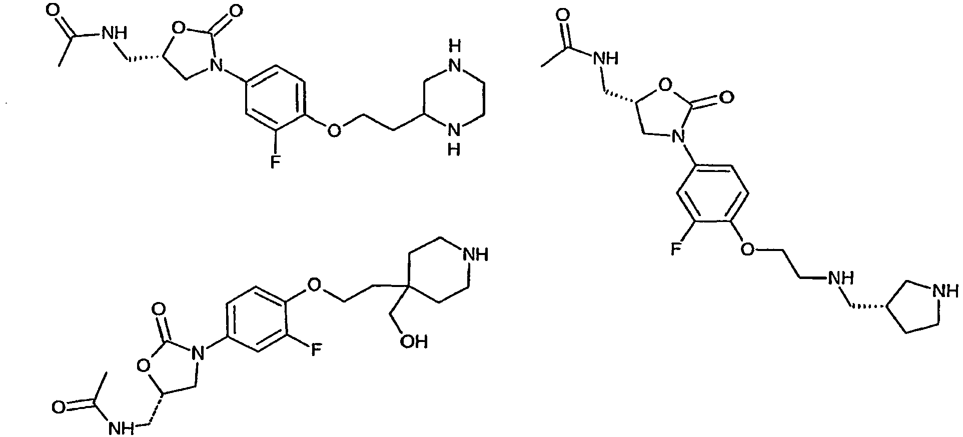

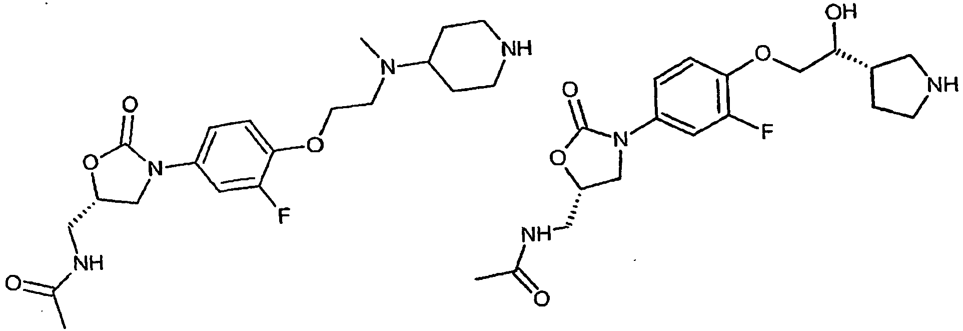

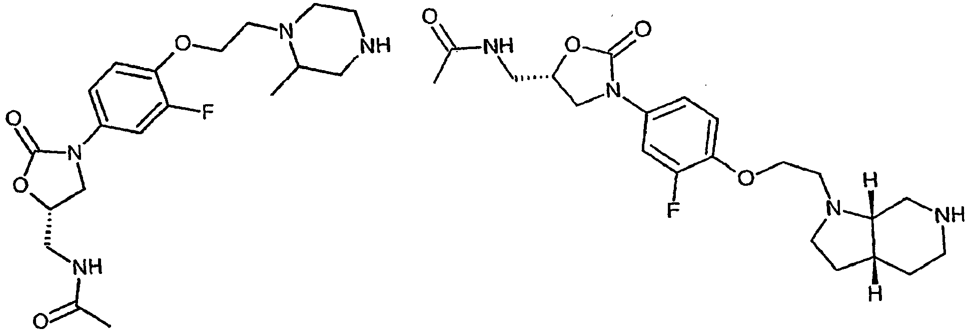

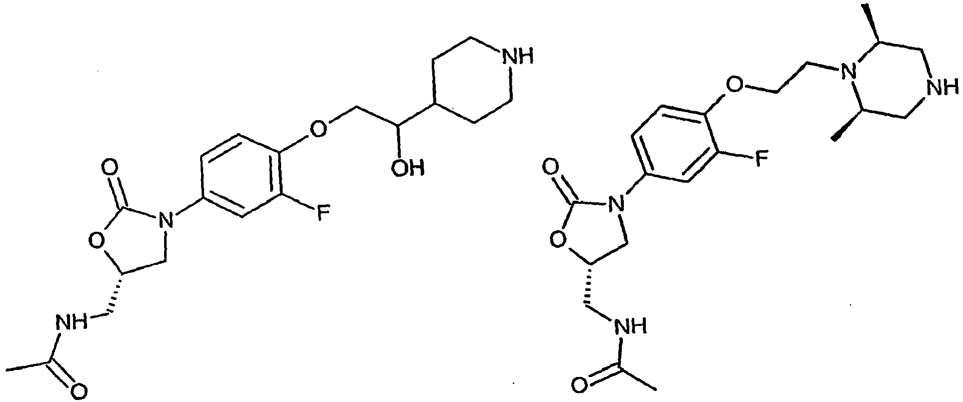

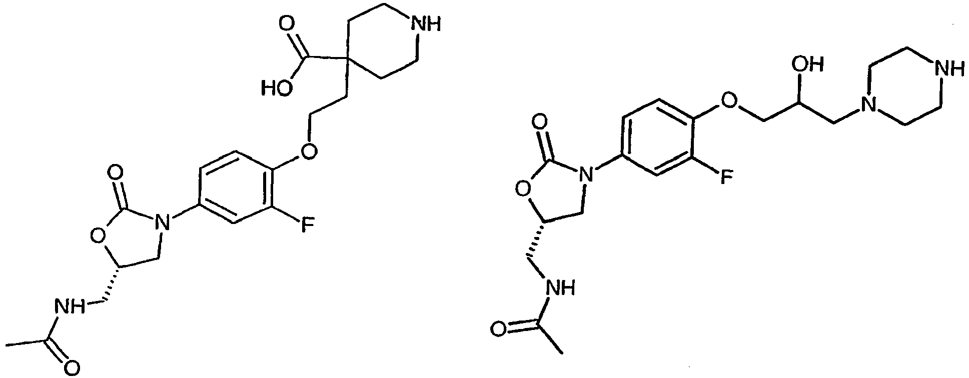

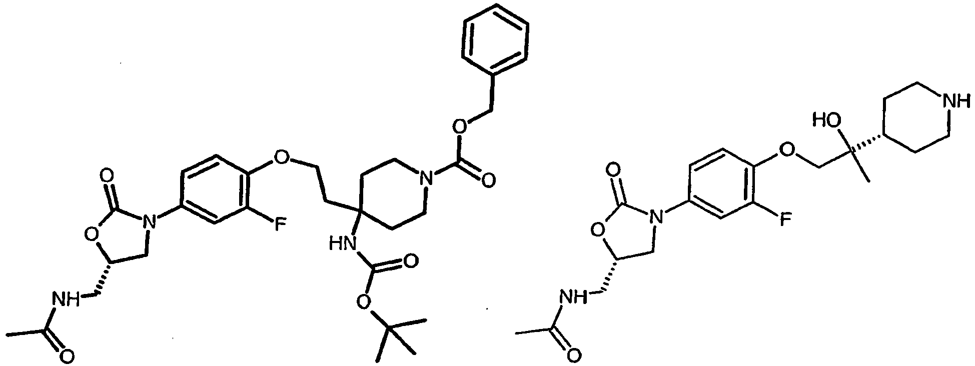

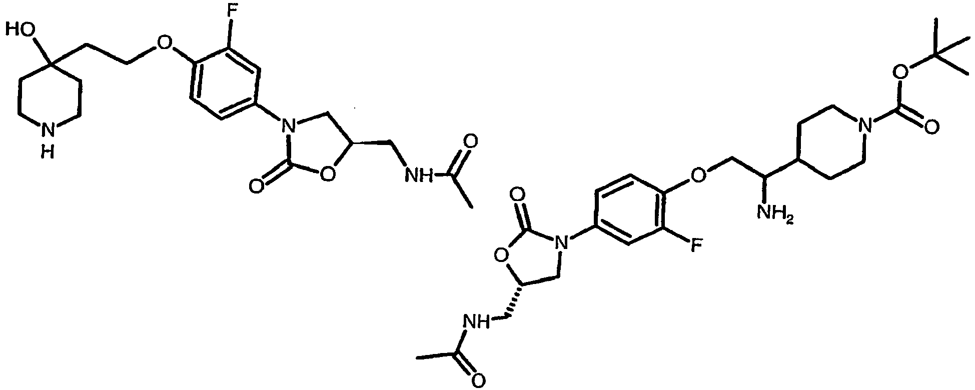

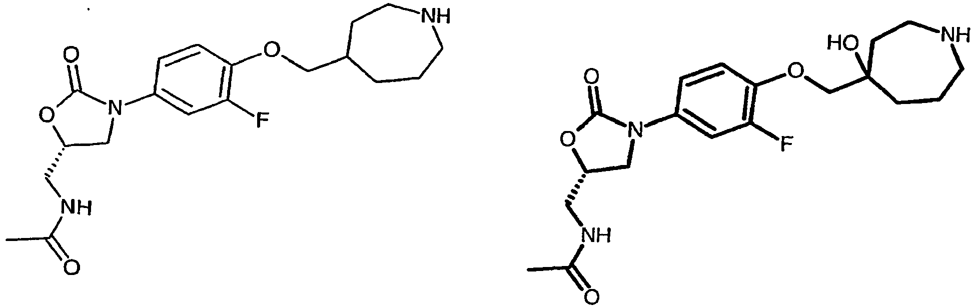

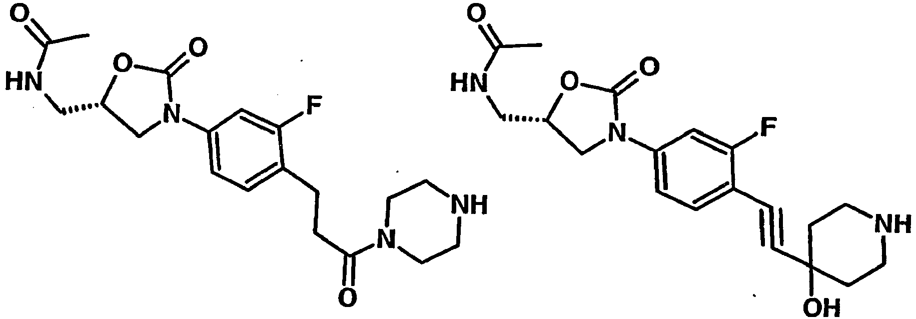

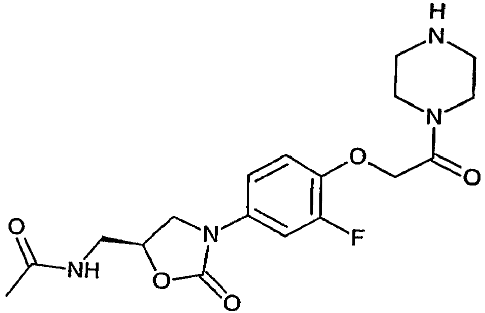

## Patentansprüche

1. Verbindungen der Formel (XI), 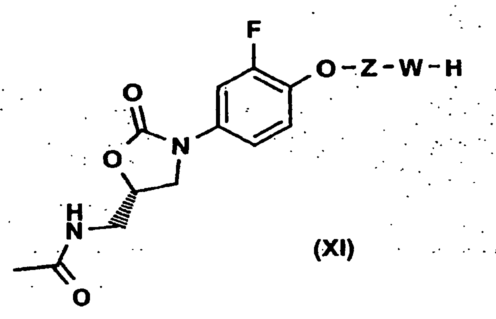 wobei
W eine durch eine OH-Gruppe substituierte Heterocycloalkylengruppe ist, die einen Ring mit 4, 5, 6 oder 7 Ringatomen enthält und wobei das an die Gruppe W gebundene Wasserstoffatom an ein Stickstoffatom gebunden ist; und
Z eine C₁₋₄ Alkylengruppe ist.

2. Verbindungen nach Anspruch 1, wobei Z eine CH₂ oder eine CH₂CH₂ Gruppe ist.

3. Verbindungen nach Anspruch 1, wobei Z-W zusammen eine Gruppe der Formel 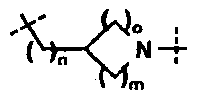 sind, wobei n gleich 1 oder 2, m gleich 1 oder 2 und o gleich 1 oder 2 ist, wobei diese Gruppe durch eine OH-Gruppe substituiert ist.

4. Verfahren zur Herstellung einer Verbindung der Formel (I): 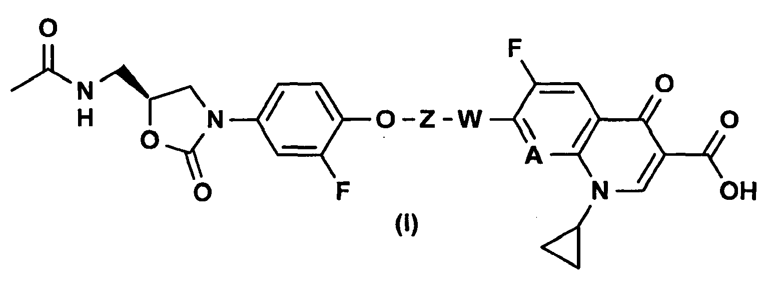 wobei Z und W wie in einem der Ansprüche 1 bis 3 definiert sind und A ein Stickstoffatom oder eine CH-Gruppe ist, **dadurch gekennzeichnet, dass** eine Verbindung der Formel (XI) nach einem der Ansprüche 1 bis 3 mit einer Verbindung der Formel (XII) nach folgendem Schema umgesetzt wird:

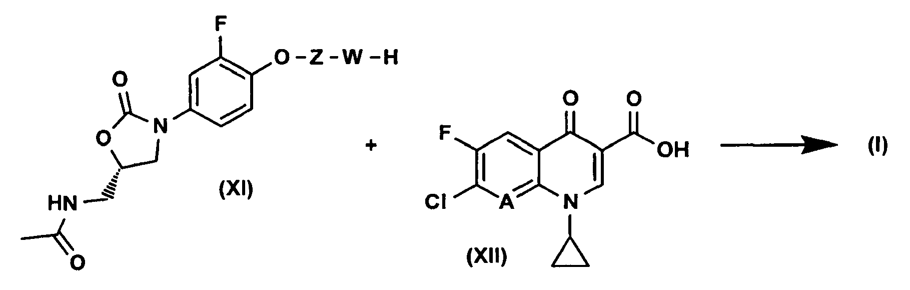

## Claims

1. Compounds of formula (XI), 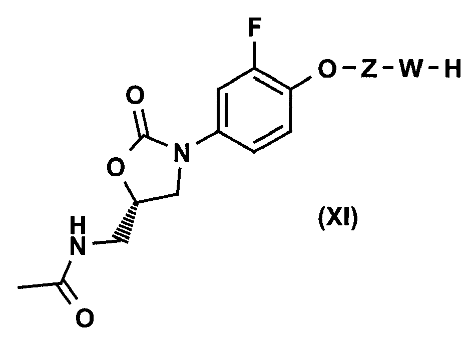 wherein
W is a heterocycloalkylene group which is substituted by an OH group and which contains a ring having 4, 5, 6 or 7 ring atoms, and wherein the H atom bonded to group W is bonded to a nitrogen atom; and
Z is a C₁₋₄ alkylene group.

2. Compounds according to claim 1, wherein Z is a CH₂ or a CH₂CH₂ group.

3. Compounds according to claim 1, wherein Z-W together are a group of formula 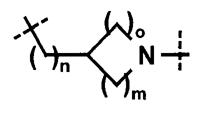 wherein n is 1 or 2, m is 1 or 2 and o is 1 or 2, wherein that group is substituted by an OH group.

4. Process for the preparation of a compound of formula (I): 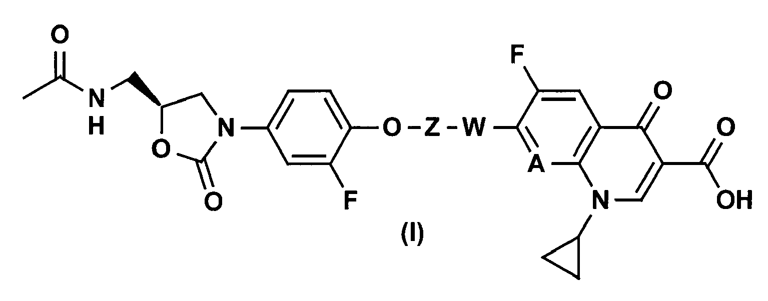 wherein Z and W are defined as in one of claims 1 to 3 and wherein A is a nitrogen atom or a CH group,
**characterized in that** a compound of formula (XI) according to any one of claims 1 to 3 is reacted with a compound of formula (XII) according to the following scheme:

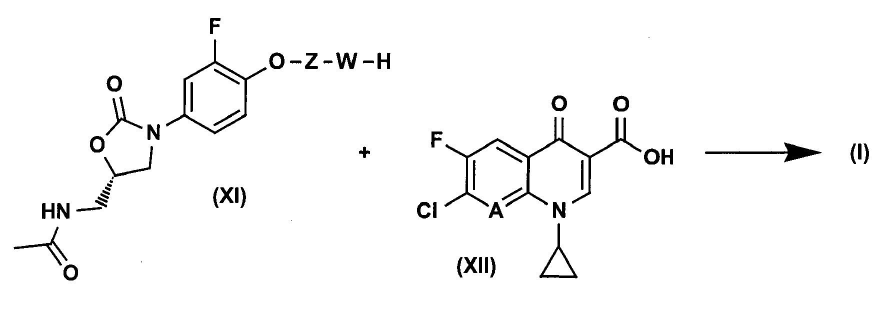

## Revendications

1. Composés de la formule (XI), 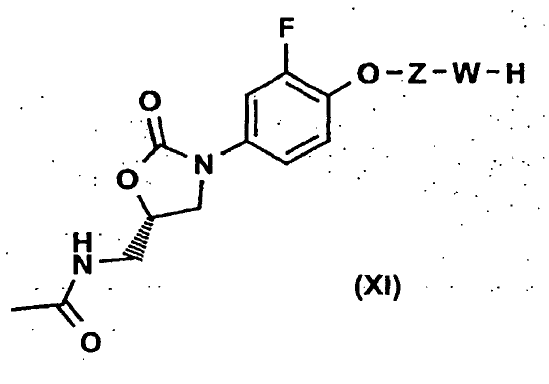 sachant que
W est un groupe hétérocycloalkylène substitué par un groupe OH, lequel groupe comporte un cycle doté de 4, 5, 6 ou 7 atomes de cycle, et sachant que l'atome d'hydrogène lié au groupe W est lié à un atome d'azote ; et
Z est un groupe alkylène en C₁-C₄.

2. Composés selon la revendication 1, sachant que Z est un groupe CH₂ ou un groupe CH₂CH₂.

3. Composés selon la revendication 1, sachant que Z-W sont conjointement un groupe de la formule 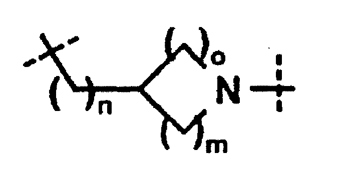 sachant que n est égal à 1 ou à 2, m est égal à 1 ou à 2 et que o est égal à 1 ou à 2, sachant que ledit groupe est substitué par un groupe OH.

4. Procédé servant à fabriquer un composé de la formule (I) : 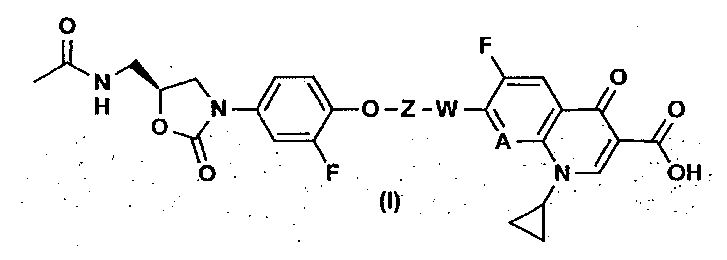 sachant que Z et W sont tels que définis dans l'une quelconque des revendications 1 à 3 et que A est un atome d'azote ou un groupe CH, **caractérisé en ce qu'**on fait réagir un composé de la formule (XI) selon l'une quelconque des revendications 1 à 3 avec un composé de la formule (XII) selon le schéma qui suit :
